# EUROPEAN PATENT SPECIFICATION

(11) **EP 1 220 826 B1**
(45) Date of publication and mention of the grant of the patent: **22.09.2004**
(21) Application number: 00957329.6
(22) Date of filing: 08.08.2000
(51) Int. Cl.: C07C 51/41, C07C 51/42, C07C 63/38, C07C 63/15

(54) **PROCESS FOR THE RECOVERY OF DISPROPORTIONATION REACTION PRODUCTS**
VERFAHREN ZUR RÜCKGEWINNUNG VON DISPROPORTIONIERUNGSREAKTIONSPRODUKTEN
PROCEDE DE RECUPERATION DE PRODUITS DE REACTION DE DISMUTATION

(30) Priority: 30.08.1999 US 151498 P
(43) Date of publication of application: 10.07.2002
(73) Proprietor: Mossi & Ghisolfi International S.A., 1528 Luxembourg (LU)
(72) Inventor: CHEN, Ye-Mon, Houston, TX 77479 (US); SALTER, James, A., Katy, TX 77450 (US)
(74) Representative: Gerbino, Angelo
(86) International application number: PCT/US2000/021661
(87) International publication number: WO 2001/016078

(56) References cited:
- US-A- 3 751 457
- US-A- 3 952 052

## Description

### Technical Field

This invention is related to a process for separating solids and purification in an integrated process for producing 2,6-naphthalene dicarboxylic acid. More particularly, the invention is related to the recovery of solid reaction products after a reaction, such as, for example, a disproportionation or rearrangement reaction in a process for making 2,6-NDA. This invention provides an improved method for separating the solids exiting the disproportionation reactor from the reaction medium that is more reliable and effective than filtration or other methods known in the art. This invention maximizes solids recovery and recycle of catalysts and dispersant while using the minimum amount of energy added to the process. It also makes it possible to maximize the yield of the aromatic carboxylic acid, such as 2,6-NDA in the overall process.

### Background Art

Processes for producing aromatic carboxylic acids are known in the art. Much work has been focused in particular on the production of 2,6-naphthalene dicarboxylic acid, because there is such a demand for it in the synthesis of polymeric materials. Some methods involve oxidation. See, for example, U.S. Patent Nos. 2,833,816; 3,870,754; 4,933,491; and 4,950,786. Other processes utilize the step of disproportionation. It is known in the art that naphthalene monocarboxylic acid and naphthalene dicarboxylic acids other than 2,6-naphthalene dicarboxylic acid can be converted to 2,6-NDA using a disproportionation reaction in the case of the monocarboxylic acids or a rearrangement reaction in the case of other napthalene dicarboxylic acids. Henkel and Cie first patented a reaction of naphthoic acid salts to 2,6-NDA in the late 1950s. (See U.S. 2,823,231 and U.S. 2,849,482). After a disproportionation or rearrangement reaction the dialkali metal salts have to be separated and converted into the desired 2,6-NDA.

There have been different approaches to the separation of the dialkali metal salt products of disproportionation reactions and conversion of them into 2,6-NDA.

In U.S. 2,828,231, the method used to separate the dialkali metal salts of 2,6-naphthalene dicarboxylic acid comprises dissolving the disproportionation conversion product mixture in water, filtering off insoluble impurities from the resulting solution, acidifying the filtrate with mineral or organic acid, such as sulfuric or hydrochloric acid, and separating the precipitated naphthalene-2,6-dicarboxylic acid from the acid solution. In U.S. 2,823,231 the dialkali metal salt of naphthalene 2,6-dicarboxylic acid formed is converted into free naphthalene 2,6-dicarboxylic acid by acidification of said di-alkali metal salt with a strong mineral acid.

U.S. 2,849,482 teaches acidifying an aqueous solution of the crude reaction product of the disproportionation or converting the crude alkali metal salt directly into the dichloride or into esters of -2,6-naphthalene dicarboxylic acid in accordance with known methods.

In U.S. 3,631,096, to Phillips, salts formed by the reaction can be transformed into the corresponding free acids by acidifying the solution with organic or inorganic acids or by introducing carbon dioxide into the solution at atmospheric or elevated pressure, and then separating the free acids from the acidified solution. The individual reaction products may be separated from each other and isolated in pure form by methods that are based upon their different solubilities or volatilities and may thereafter, if desired, be transformed into their derivatives. The salt mixture produced by the reaction may also be transformed directly into derivatives of the acids, for example, into their esters or halides, and these derivatives may be purified, if desired, by fractional distillation.

U.S. 3,671,578, to Teijin, discloses that the monoalkali salt of 2,6-naphthalene dicarboxylic acid is easily disproportionated when heated in water or water-containing organic solvent, to form free dicarboxylic acid and by-product dialkali salt, and the former acid is precipitated.

U.S. 3,888,921, to Teijin Ltd., discloses a method for purifying a dialkali salt of crude 2,6-naphthalene dicarboxylic acid comprising precipitating 40 to 97 mol percent of the dialkali 2,6-naphthalene dicarboxylate dissolved in an aqueous solution substantially as a monoalkali salt of the 2,6-naphthalenedicarboxylic acid while maintaining the pH of said aqueous solution at a value not lower than 6.3, and separating the precipitate, and converting the separated precipitate to a 2,6-naphthalene dicarboxylic acid.

Canadian Patent 864587 discloses a process for the preparation of 2,6-NDA which comprises heating a monoalkali salt of 2,6-NDA in water or water-containing organic solvent causing disproportionation thereof into 2,6-NDA and a dialkali salt and separating the 2,6-NDA by a method that includes dissolving a rearrangement reaction product containing dialkali salt of 2,6- naphthalene dicarboxylic acid in warm water, filtering off the insoluble matter therefrom, concentrating the remaining solution, whereby the filtrate is concentrated to such a degree that the precipitation yield of the dialkali salt precipitated when the concentrated liquid is cooled to room temperature reaches at least 70% and the purity of said precipitate exceeds 99%, passing gaseous carbon dioxide through the aqueous solution of the precipitate recovered from the concentrated liquid, and recovering the resulting precipitate,and the mother liquour containing the side product dialkali salt of 2,6-naphthalene dicarboxylic acid is recycled into the carbon dioxide reaction step.

Various methods for separating the reaction medium from the aromatic dicarboxylic acids are disclosed in the art. For example, benzene extraction, evaporation, and sublimation are known. Water extraction is vulnerable to problems which are often associated with the settling and phase separation of large amounts of aqueous solutions mixed with large amounts of organic solutions. Evaporation or sublimation, on the other hand, is a slow and costly method, both with respect to the apparatus and to the energy necessary.

As noted, many of these methods rely on filtration at least in part to separate the dipotassium salt products of disproportionation, but filtration is expensive and unreliable. An improved method of separating the disproportionation solids from the remainder of the effluent materials would ultimately allow for better conversion and yield of the desired 2,6-NDA.

In U.S. 3,952,052, to Phillips, there is disclosed a process for separating a disproportionation reaction product by forming a slurry, comprising alkali metal salts of aromatic polycarboxylic acid and dispersant, and mixing the slurry with the gaseous effluent, and then lowering the pressure, flashing the dispersant, and recovering said alkali metal salts of said polycarboxylic acids as solids from said separation zone.

The instant invention, while somewhat similar to U.S. '052, provides significant improvement in overall reliability and cost, which will be discussed in the

### Detailed Description of the Invention.

### Disclosure of the Invention

In accordance with the foregoing the present invention is a process for separating disproportionation reaction product solids according to claim 1. Prefered features of the invention are disclosed in the dependent claims.

### Detailed Description of the Drawings

Figure 1 is a flow diagram showing the preferred embodiment.
Figure 2 shows the details of the fluidic valve and the pressure letdown pipe.
Figure 3 is a flow diagram showing in the prior art of U.S. 3,952,052.

### Detailed Description of the Invention

The disproportionation reactor product solids to be separated in the present invention can comprise any alkali metal salt of an aromatic dicarboxylic acid along, with unreacted feed, catalyst, and trace coke.

These alkali metal salts are intermediates in the production of aromatic dicarboxylic acids, which are useful in the production of various polymers. Examples include alkali metal salts of terephthalic acid and naphthalene dicarboxylic acid. A particularly desirable aromatic dicarboxylic acid is 2,6-naphthalene dicarboxylic acid, because of its superior properties when reacted with ethylene glycol to form poly(2,6-ethylene naphthalate). The dipotassium salt of 2,6-naphthalene dicarboxylic acid is a precursor of 2,6-naphthalene dicarboxylic acid formed by a process that incorporates a disproportionation reaction.

The feed for the disproportionation reaction is an alkali salt of a mono- or dicarboxylic acid which is changed by the disproportionation reaction to an isomer of a dialkali salt of 2,6-NDA with injection of carbon dioxide. The alkali metal salts of the aromatic mono- or dicarboxylic acids are introduced into a disproportionation reactor along with a dispersant, and a catalyst. This is typically part of a larger integrated process, as described in copending U.S. Ser. No. 60/151,498, filed of even date. Said alkali metal salts of mono- or dicarboxylic acids, carbon dioxide and catalyst are reacted at elevated temperature and high pressure and the effluent of the reactor is a slurry containing said alkali metal salts of aromatic dicarboxylic acids, unreacted feed, catalyst, trace coke, and dispersant.

The reaction medium can be any dispersant with sufficient thermal stability. It is not restricted to aromatic compounds, however aromatic compounds are suitable. Examples of suitable dispersants include a single compound or mixture of compounds selected from a variety of aprotic polycyclic aromatic compounds, such as, for example, naphthalene, methylnaphthalene, dimethylnaphthalene, diphenyl ether, dinaphthyl ether, terphenyl, anthracene, phenanthrene, and mixtures thereof. The polycyclic aromatic compound is used in an amount of 1 to 6 times, preferably 2 to 4 times, the amount of the starting material based on weight.

In the preferred embodiment of the present invention the alkali salt is potassium naphthoate which is introduced into the disproportionation reactor along with napthalene as a dispersant and a catalyst comprising a zinc compound, such as zinc oxide or zinc napththoate. Carbon dioxide is also fed into the reactor.

Typical operating conditions for the disproportionation reactor are disclosed in U.S. Pat. Nos. 3,751,457 and 3,781,341. Temperatures in the range of 420°C to 460°C and pressures of 4826 to 8274 kPa (700 to 1200 psia) are preferred. The residence time of the components in the disproportionation reactor is 0.3 to 1 hour. The dispersant, e.g. naphthalene, is present in the reaction slurry in an amount of 25 to 80% by total weight of the slurry.

The disproportionation reaction is carried out either batchwise or continuously in any suitable reactor or sequence of reactors in which the desired temperatures and pressures can suitably be maintained and which can adequately be used for charging, stirring, and discharging of slurries.

The hot effluent slurry from the disproportionation reactor comprises disproportionated alkali metal salts of dicarboxylic acids, and dispersant, together with small amounts of other materials. In the case where potassium naphthoate is introduced into the disproportionation reactor, the effluent comprises isomers of the dipotassium salt of 2,6-NDA (2,6 K2NDA and 2,3 K2NDA), unreacted KNA, catalyst and trace coke. Said effluent is in a liquid/solid slurry. In the present invention the liquid/solid slurry exits the disproportionation reactor where the pressure is in the range of 4137 to 10342 kPa (600 to 1500 psi), preferably 5516 to 8274 kPa (800 to 1200 psi), with the most preferred pressure 5516-6550 kPa (800-950 psi).

The following description of the preferred embodiment in connection with the drawing will enable a person skilled in the art to better understand and practice the invention. The drawing is described in terms of preparing the dipotassium salt of 2,6-NDA using naphthalene as a reaction medium. The specific details given are, however, not intended to limit the scope of the invention. In the drawing many details of the apparatus, such as valves, pumps, controlling means, etc have been omitted. These items, however, are well known to one skilled in the art. Any suitable apparatus of this nature can be used.

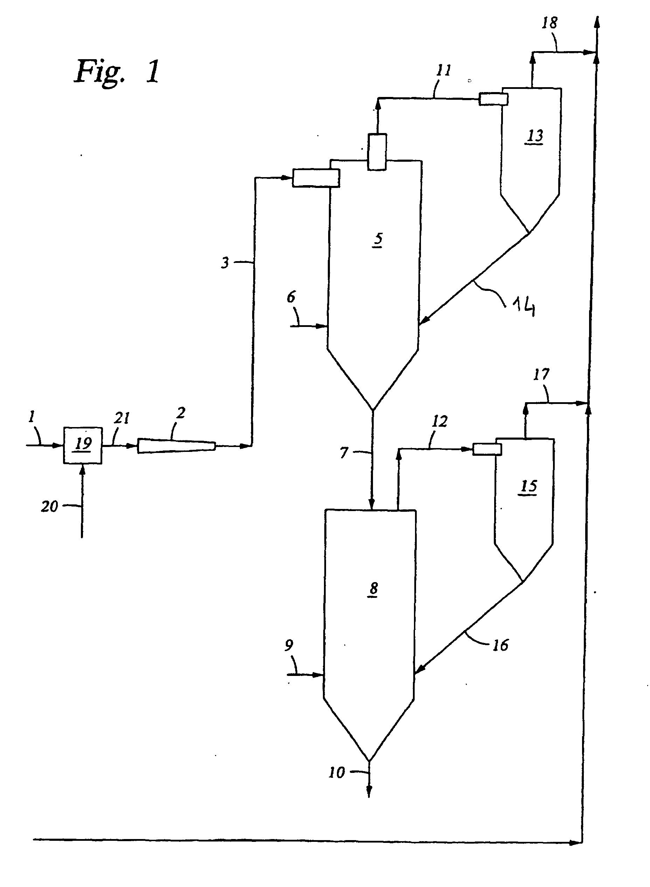

Referring now to Figure 1, the effluent slurry of solids in naphthalene, consisting of 2,6 K2NDA, 2,3 K2NDA(isomer intermediate), unreacted KNA, catalyst, and trace coke exits the disproportionation reactor in line 1 and enters a fluidic control valve **19** which has a second inlet of hot naphthalene **20** to control the product slurry flow rate. The combined slurry **21** then enters a pressure letdown pipe **2** through which the pressure is reduced, leading to gradual flashing of all liquid naphthalene and CO₂, thus converting a liquid/solid slurry **21** to a gas/solid mixture that exits in line **3**. The mixture in **3** first enters a stripper cyclone **5** where most of solids in the gas/solid mixture are separated and kept in the lower part of **5** for stripping. Naphthalene and CO₂ vapor with some entrained solids then exit at **11** and enter a second stage cyclone **13.** Additional solids are separated in **13** and returned to the lower part of the stripper cyclone **5** via **14** for stripping.Naphthalene and CO₂ vapor exit **13** at **18**. The solids in the lower part of **5** are stripped with CO₂ via **6**. The stripped solids exit **5** in line **7** and enter the second stage stripper **8** where additional stripping gas of CO₂ is introduced via 9. The stripping gas and entrained solids exit **8** at **12** and enter cyclone **15** where most entrained solids are separated and returned to stripper **8** via **16.** The stripped K2NDA solids exit at **10.** The stripping gas exits **15** at **17** which combines with **18** to become the recycled naphthalene and CO₂ stream.

The naphthalene and CO₂ vapor **18** exiting the pressure letdown section is recycled, used to evaporate water from the aqueous salt of naphthoic acid, and used as a carrier and dispersant in the disproportionation reaction as discussed in copending Ser. No. 60/151,577. The net naphthalene produced by the disproportionation reaction is either condensed for production of additional methylnaphthalene via transalkylation with dimethylnaphthalene or sold to the merchant market.

Following the separation and stripping, the solid product **10** comprising dipotassium salts of NDA, K2NDA (2,6-and 2,3-isomers), unreacted KNA, catalyst, heavy by-products, trace coke and entrained CO₂ vapor are washed, followed by a series of steps for isolation and purification of 2,6-NDA as described in copending U.S. Ser. No. 60/151,577. The solids are contacted with water to create a mixture of aqueous potassium salts comprising potassium naphthoate, and the dipotassium salt of 2,6-naphthalene dicarboxylic acid and its isomers, and solid catalyst. This mixture is filtered to separate the solid catalyst which is recycled. Then aqueous potassium bicarbonate is added to the mixture of aqueous potassium salts and a portion of the water is evaporated to selectively crystallize the dipotassium salt of 2,6-NDA as a solid. The solid dipotassium salt of 2,6-NDA is dissolved in water and optionally passed through an activated carbon bed to remove impurities. The aqueous dipotassium salt of 2,6-NDA is contacted with carbon dioxide to create a mixture of the solid monopotassium salt of 2,6, -NDA and aqueous potassium bicarbonate. The monopotassium salt of 2,6-NDA is contacted with water, optionally in the presence of CO₂, to form solid 2,6-NDA, aqueous dipotassium salt of 2,6-NDA, and potassium bicarbonate. The solid 2,6-NDA is contacted with water in a pipe reactor to remove traces of potassium ion impurity.

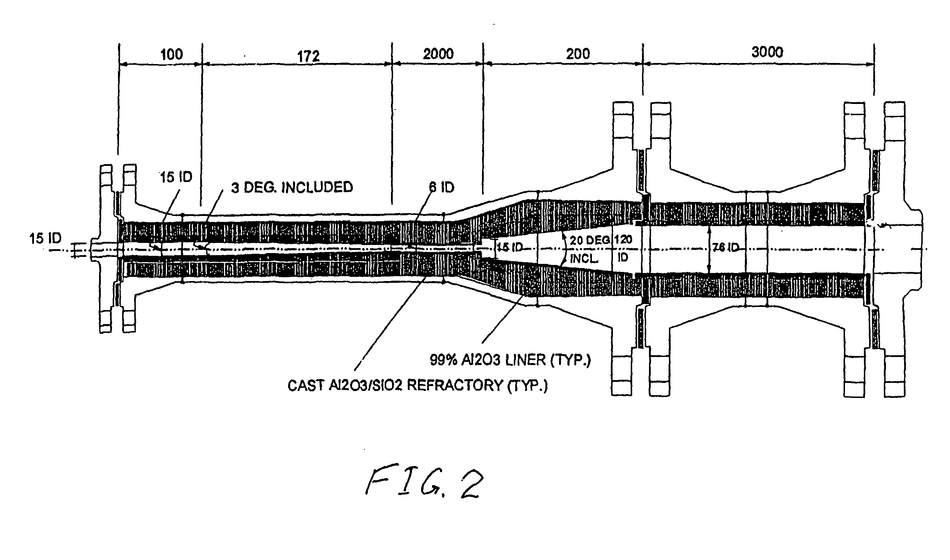

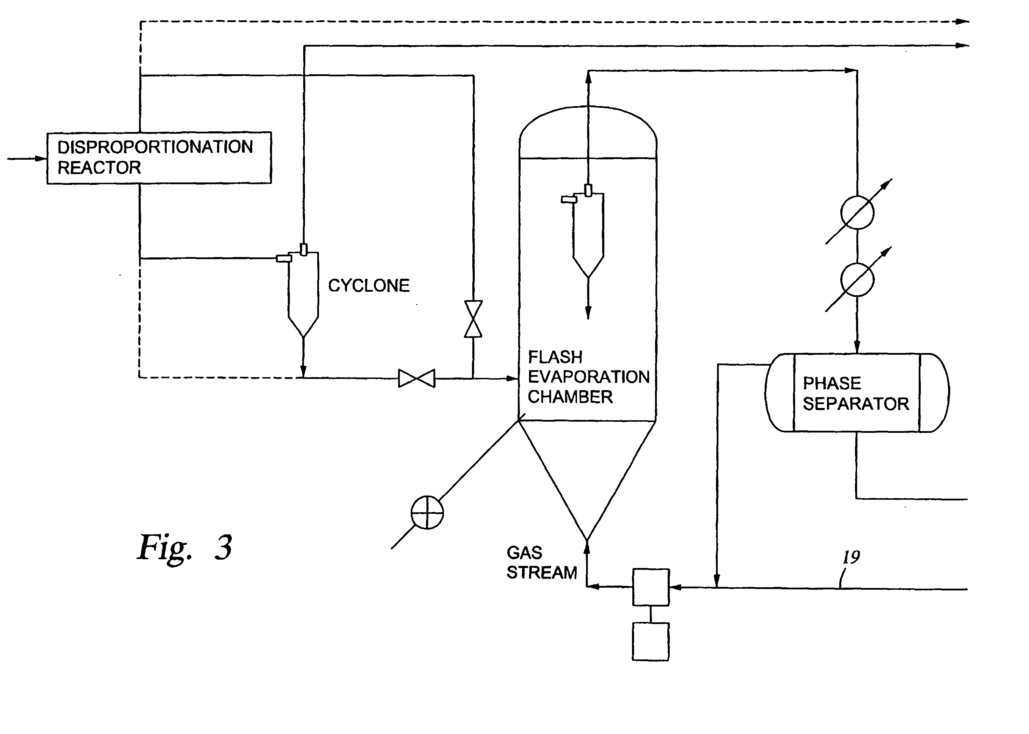

One key element in the present invention is the control of pressure letdown of the effluent slurry from the disproportionation reactor. Figure 2 shows the details of the fluidic valve **19** and the pressure letdown pipe **2** in the preferred embodiment of Figure 1. The fluidic valve is shown as a simple tee, which can be one of several different types known in the art, with two inlets; one for disproportionation product slurry **1** and the other for hot naphthalene **20.** The combined steam **21** exits the fluidic valve to feed the pressure letdown pipe **2** through which the pressure is gradually reduced. The result of the pressure letdown is that all naphthalene in the combined liquid/solid slurry **21** is gradually flashed and the liquid/solid slurry is changed to gas/solid mixture **3** at the outlet with no additional heat input.

The combination of the fluidic control valve **19** and the pressure letdown pipe **2** controls the flow rate of the disproportionation reactor products stream **1** without the need of a mechanical control valve, which is unreliable in highly erosive slurry service. The control mechanism of the fluidic valve is based on the fact that the letdown pipe **2** can only flush a certain amount of naphthalene at a given dimension and pressure differential. By increasing the flow rate of hot naphthalene **20** into the fluid control valve **19,** the flushing rate of naphthalene through the letdown pipe **2** remains constant, but the flow rate of the disproportionation reactor products **1** can be reduced to any amount, including no flow.

Referring to Figure 2 for details of the pressure letdown.pipe **2**, the tapered bore can be one of several different types known in the art. The pipe is shown to have a tapered bore of pipe section with decreasing diameter where the liquid/solid mixture is accelerated, causing pressure to drop and part of the liquid to flash, which leads to increased acceleration, a pressure drop and additional liquid flashing. Generally the letdown pipe should have:
a) a straight pipe section having a length of at least 20 pipe diameters with an opening at the end for receiving the liquid/solid phase product exiting the disproportionation reactor and an opening at the other end for discharging the liquid/solid phase product;
b) a concentric tapered bore converging in the direction of flow with an inlet diameter identical to the preceding straight section, an included angle of 3° or less and an outlet diameter identical to that of the following straight section;
c) a concentric straight bore section having an inside diameter and length extending in the direction of its longitudinal axis selected to produce the required pressure drop over the range of operation;
d) a concentric, tapered bore section diverging in the direction of flow with an included angle of not more than 14°; and
e) terminating in a pipe or line which receives the resulting gas/solid phase product and transports said gas/solid into the first stripper cyclone.

Another key element of the present invention is the use of stripper cyclone to strip out naphthalene from the solids products. The stripper cyclone in the present process is similar to that disclosed in U.S. 4,455,220, but preferably comprises two stages of stripping, as shown in Figure 1.

This method is allows for maximizing the recovery of solids which, in turn, contributes to improved yields of 2,6-naphthalene dicarboxylic acid in subsequent steps where the 2,6-naphthalene dicarboxylic acid is isolated and purified.

From the description above, it becomes clear that the present invention provides significant improvements in overall reliability and cost, compared to U.S. 3,952,052 which is shown in Figure 3. Distinctions are as follows:
- **Handling of effluent gas -** In '052 the product slurry from the disproportionation reactor is combined with the effluent gas before lowering the pressure. In the instant invention, the effluent gas from the disproportionation reactor is treated separately, which makes the separation equipment more compact, permitting lower capital cost.
- **Means of pressure letdown and flow control -** In '052, "the separation zone can comprise any suitable flash evaporation chamber and related means by which feeds are sprayed into a chamber which is maintained at a reduced pressure." (column 3, Line 48 to 52). Two major improvements in the present invention are that the combination of **a fluidic valve and a pressure letdown pipe**, instead of a spray, is used as the means of pressure letdown and flow control, and **no flashing chamber is required**. We have discovered in coal gasification process development that a sudden pressure letdown using a control valve or spray nozzle, as in '052, cannot last for more than a few days or weeks of continuous operation with fluid containing substantial amount of erosive particles. The control valve or spray nozzle will be so severely eroded that it can no longer control the pressure letdown and has to be replaced. The renders the system taught in '052 highly unreliable. In the present invention, pressure reduction occurs more gradually in a section of tapered pipe, instead of sudden pressure reduction through a valve or spray, thus significantly reducing the severity of erosion. We have demonstrated in coal gasification process development (TPR WTC 72-96 Shell Coal Gasification Process - Equipment Development - Fly Ash Letdown Bench Scale Test Facility; TPR WRC 26-93 Shell Coal Gasification Process - Equipment Development - Fly Ash Letdown - Field Test:; 4,838,898 Method for Removal and Disposal of Fly Ash from a High-Temperature, High-Pressure Synthesis Gas Steam; 4,877,419 Stripping and Depressurization of Solids and Gas Mixture) that the pressure letdown pipe can last for more than 6 months in highly erosive pressure letdown operation which significantly improves the reliability of the separation system. We have also demonstrated that the fluidic valve can control the slurry flow over the same period. Another improvement is that the present invention does not require a flashing vessel. The first stripper cyclone is used as the receiving vessel after the pressure letdown, which makes the system much more compact, compared to '052, thus reducing capital cost.
- **Product stripping -** In '052 "The gas stream introduced into the flash evaporation chamber exerts a fluidizing action upon the solids accumulated in the lower portion of said chamber. This fluidizing action prevents the solids from caking together and enables continuous removal of the solid products for the process". The gas stream consists of carbon dioxide, benzene and terphenyl in '052'. From the above description, it is clear that in '052 the gas stream introduced into the flush chamber is only for fluidization purpose to assist product removal. A major disadvantage of this system is that hydrocarbon vapor, such as benzene and terphenyl, will be discharged when the solids are removed from the flashing chamber which significantly complicates the downstream product purification process. In the instant invention, two stages of stripping using only carbon dioxide to remove all hydrocarbon vapors, such as naphthalene from solid products. This improvement significantly reduces downstream separation cost, because only carbon dioxide gas is removed with the solids in the subsequent step of dissolving the product solids in water.

The following example is provided to illustrate the present invention as applied to the production of dipotassium naphthoate using naphthalene as. a reaction medium and zinc oxide as catalyst in an apparatus such as that schematically shown in the drawing.

### EXAMPLE

Potassium napthoate and zinc oxide are introduced together with naphthalene and carbon dioxide into a disproportionation reactor. The disproportionation reaction is carried out at 5516 kPa (800 psia) and 460°C. The slurry comprising 2,6 K2NDA, 2,3 K2NDA(isomer intermediate), unreacted KNA, catalyst, trace coke and naphthalene exits the disproportionation reactor where the pressure is about 5516 kPa (800 psi) and enters a fluidic valve with hot naphthalene injection, followed by a tapered bore of decreasing diameter where the liquid/solid mixture is accelerated, causing pressure to drop and part of the liquid to flash, which leads to increased acceleration, a further pressure drop and liquid flashing. The result of the pressure letdown is that all naphthalene is flashed gradually and the liquid/solid slurry becomes gas/solid mixture. This gas solid mixture is directed to two stages of stripping and cyclone separation, as described in the detailed description of the drawing.

## Claims

1. In a process for producing aromatic dicarboxylic acids which includes a disproportionation reaction of an alkali salt of mono- or dicarboxylic acid in a liquid reaction medium to produce an effluent containing reaction medium and disproportionation reaction solids comprising isomers of the alkali salt of the product dicarboxylic acid, unreacted feed, catalyst and trace coke, all of the aforesaid exiting said disproportionation reactor as a liquid/solid slurry, an improved process for separating the disproportionation product solids from the reaction medium, which comprises:
a) directing said reactor effluent in the form of liquid/solid slurry through a fluidic valve using a second injection of liquid reaction medium to control the flow of reactor effluent,
b) thereafter directing said reactor effluent, without the addition of effluent gas into a pressure letdown section comprising a section of tapered pipe where the liquid/solid slurry is accelerated, causing pressure to drop and the liquid to flash,
c) thereby gradually flashing all liquid from the reactor effluent slurry in the pressure letdown section, resulting in a change in said reactor effluent from liquid/solid slurry to gas/solid mixture;
d) directing said gas/solid mixture into at least one stripper cyclone where solids are separated and stripped using using only carbon dioxide, and gases exit and are recycled, and thereafter
e) recovering said solids comprising isomers of the alkali salt of the product dicarboxylic acid, unreacted feed, catalyst and trace coke for further purification.

2. The process of Claim 1 wherein said aromatic dicarboxylic acid is 2,6-naphthalene dicarboxylic acid, said solids exiting the disproportionation reactor comprise the isomers dipotassium salts of 2,6- and 2, 3 naphthalene dicarboxylic acid, unreacted potassium naphthoate, catalyst and trace coke, and the reaction medium is naphthalene.

3. The process of Claim 1 wherein the liquid/solid slurry exiting the disproportionation reactor is at a pressure above 4481 kPa (650 psi).

4. The process of Claim 1 wherein in step (a) the fluidic valve is a tee.

5. The process of Claim 1 wherein in step (a) the second injection of the reaction medium is naphthalene.

6. The process of Claim 1 wherein in step (a) said the flow control of reactor effluent is achieved in the absence of mechanical means.

7. The process of Claim 1 wherein a separate flashing chamber is not required.

8. The process of Claim 1 wherein, after flashing all of liquid reaction medium, the gas/solid mixture is received directly into a receiving cyclone.

9. The process of Claim 1 wherein carbon dioxide gas can be removed by dissolving the product solids in water.

10. The process of Claim 2 wherein all naphthalene is flashed with no additional heat input.

11. The process of Claim 1 wherein there are two stages of strippers.

12. The process of Claim 11 wherein the first stage 12 stripper and the receiving cyclone are combined into a single vessel of stripper cyclone.

13. The process of Claim 12 wherein the first stripper is situated above the second.

14. The process of Claim 13 wherein CO2 gas is introduced at the bottom of each stripper.

15. The process of Claim 14 wherein CO2 and some entrained solids exit the strippers and enter respectively attached secondary cyclones where additional solids are separated.

16. The process of Claim 15 wherein the secondary cyclones are equipped with a means of returning additional separated solids to the respective strippers.

17. The process of Claim 16 wherein said strippers, one above the other, and attached secondary cyclones, each with a means of returning separated solids to the strippers to which it is attached, allow for maximum recovery of solids, and the combined solids exit the second stripper.

18. The process of Claim 3 wherein said solids exiting said disproportionation reactor in a liquid/solid slurry are at a pressure in the range of 5171 to 6550 kPa (750 to 950 psi).

19. The process of Claim 2 wherein said solid product comprising dipotassium salts of naphthalene dicarboxylic acid, the dipotassium salts of 2, 6- and 2, 3 naphthalene dicarboxylic acid, unreacted KNA, catalyst, heavy by-products, and trace coke exit the last stripper and are directed to a water wash where the organic salts are dissolved.

20. The process of Claim 2 wherein the naphthalene vapor exiting the strippers is recycled.

## Patentansprüche

1. Verfahren zur Herstellung von aromatischen Dicarbonsäuren, wobei das Verfahren eine Disproportionierungsreaktion eines Alkalisalzes einer Mono- oder Dicarbonsäure in einem flüssigen Reaktionsmedium aufweist, um einen Ausfluss zu erzeugen, der das Reaktionsmedium und Feststoffe der Disproportionierungsreaktion enthält, die Isomere des Alkalisalzes der Produkt-Dicarbonsäure, nichtreagierte Beschickung, den Katalysator sowie Kohlenstoffspuren enthalten, wobei alle oben angeführten Stoffe aus dem Disproportionierungsreaktor als flüssig/feste Aufschlämmung austreten, wobei ein verbessertes Verfahren zum Abtrennen der festen Disproportionierungsprodukte vom Reaktionsmedium enthält:
Leiten des Reaktorausflusses in Form einer flüssig/festen Aufschlämmung über ein Fluidventil unter Verwendung einer zweiten Einspritzung eines flüssigen Reaktionsmediums, um die Strömung des Reaktorausflusses zu steuern;
daraufhin Leiten des Reaktorausflusses ohne der Beigabe eines ausfließenden Gases in einen Druckreduzierbereich, der einen Bereich mit einem konischen Rohr enthält, wo die flüssig/feste Aufschlämmung beschleunigt wird, wodurch der Druck abfällt und die Flüssigkeit abdampft;
dadurch allmähliches Abdampfen der gesamten Flüssigkeit von der aus dem Reaktor ausfließenden Aufschlämmung im Druckreduzierbereich, wodurch eine Änderung im Reaktorausfluss von der flüssig/festen Aufschlämmung in ein gasförmig/festes Gemisch entsteht;
Leiten des gasförmig/festen Gemischs in zumindest einen Stripperzyklon, in dem Feststoffe abgetrennt und nur unter Verwendung von Kohlendioxid gestrippt werden, und Gase austreten und wieder in Umlauf gebracht werden, und nachher
Wiedergewinnen der Feststoffe, die Isomere des Alkalisalzes der Produkt-Dicarbonsäure, unreagierte Beschickung, den Katalysator und Kohlenstoffspuren enthalten, für eine weitere Reinigung.

2. Verfahren gemäß Anspruch 1, wobei die aromatische Dicarbonsäure 2,6-Naphthalin-dicarbonsäure ist, die Feststoffe, die den Disproportionierungsreaktor verlassen, die Isomere von Dikaliumsalzen der 2,6- und 2,3-Naphthalin-dicarbonsäure, nichtreagiertes Kaliumnaphthoat, den Katalysator und Kohlenstoffspuren enthalten und das Reaktionsmedium Naphthalin ist.

3. Verfahren gemäß Anspruch 1, wobei die flüssig/feste Aufschlämmung, die den Disproportionierungsreaktor verlässt, unter einem Druck von mehr als 4481 kPa (650 psi) steht.

4. Verfahren gemäß Anspruch 1, wobei im Schritt (a) das Fluidventil ein T-Stück ist.

5. Verfahren gemäß Anspruch 1, wobei im Schritt (a) die zweite Einspritzung des Reaktionsmediums aus Naphthalin besteht.

6. Verfahren gemäß Anspruch 1, wobei im Schritt (a) die Strömungssteuerung des Reaktorausflusses in Abwesenheit von mechanischen Einrichtungen erreicht wird.

7. Verfahren gemäß Anspruch 1, wobei keine getrennte Flashdestillationskammer erforderlich ist.

8. Verfahren gemäß Anspruch 1, wobei nach dem Abdampfen des gesamten flüssigen Reaktionsmediums das gasförmig/feste Gemisch direkt in einen Zyklon geleitet wird.

9. Verfahren gemäß Anspruch 1, wobei Kohlendioxidgas dadurch entfernt werden kann, dass die festen Produkte in Wasser gelöst werden.

10. Verfahren gemäß Anspruch 2, wobei das gesamte Naphthalin ohne die Zugabe von zusätzlicher Wärme abgedampft wird.

11. Verfahren gemäß Anspruch 1, wobei zwei Stufen von Strippern vorhanden sind.

12. Verfahren gemäß Anspruch 11, wobei die erste Stripperstufe und der Zyklon in einen einzigen Kessel eines Stripperzyklons kombiniert sind.

13. Verfahren gemäß Anspruch 12, wobei der erste Stripper über dem zweiten angeordnet ist.

14. Verfahren gemäß Anspruch 13, wobei CO₂-Gas am Boden eines jeden Strippers eingeleitet wird.

15. Verfahren gemäß Anspruch 14, wobei CO₂ und einige mitgeführte Feststoffe die Stripper verlassen und in jeweils zugeordnete Sekundärzyklone gelangen, wo zusätzliche Feststoffe abgetrennt werden.

16. Verfahren gemäß Anspruch 15, wobei die Sekundärzyklone mit einer Einrichtung ausgestattet sind, um zusätzlich abgetrennte Feststoffe zu den entsprechenden Strippern zurückzuführen.

17. Verfahren gemäß Anspruch 16, wobei die Stripper, einer über dem anderen, und die zugeordneten Sekundärzyklone jeder mit einer Einrichtung zum Zurückführen der abgetrennten Feststoffe zu den Strippern, denen er zugeordnet ist, eine maximale Rückgewinnung der Feststoffe ermöglichen und die kombinierten Feststoffe den zweiten Stripper verlassen.

18. Verfahren gemäß Anspruch 3, wobei die Feststoffe aus dem Disproportionierungsreaktor in einer flüssig/festen Aufschlämmung unter einem Druck im Bereich von 5171 bis 6550 kPa (750 bis 950 psi) austreten.

19. Verfahren gemäß Anspruch 2, wobei das feste Produkt, das Dikaliumsalze der Naphthalin-dicarbonsäure, Dikaliumsalze der 2,6- und 2,3-Naphthalin-dicarbonsäure, nichtreagiertes Kaliumnaphthoat, den Katalysator, schwere Nebenprodukte und Kohlenstoffspuren enthält, den letzten Stripper verlässt und zu einer Waschung mit Wasser geleitet wird, in der die organischen Salze gelöst werden.

20. Verfahren gemäß Anspruch 2, wobei die aus den Strippern austretenden Naphthalindämpfe wieder in Umlauf gebracht werden.

## Revendications

1. Procédé de production d'acides dicarboxyliques aromatiques qui comprend une réaction de dismutation d'un sel alcalin d'un acide mono- ou dicarboxylique dans un milieu réactionnel liquide pour produire un effluent contenant le milieu réactionnel et des solides de la réaction de dismutation comprenant des isomères du sel alcalin de l'acide dicarboxylique, l'alimentation n'ayant pas réagi, le catalyseur et des traces de coke, tous sortant dudit réacteur de dismutation sous la forme d'une suspension liquide/solide, un procédé amélioré de séparation des solides de la dismutation du milieu réactionnel, qui comprend les étapes consistant à :
a) diriger ledit effluent du réacteur sous la forme d'une suspension liquide/solide à travers une valve fluidique, en utilisant une deuxième injection de milieu réactionnel liquide pour contrôler l'écoulement de l'effluent du réacteur,
b) diriger ensuite ledit effluent du réacteur, sans ajout d'un effluent gazeux dans une section de régulation de pression comprenant une section de conduit fuselé où la suspension liquide/solide est accélérée, provoquant une diminution de la pression et la montée du liquide,
c) faire monter graduellement tout le liquide de la suspension de l'effluent du réacteur dans la section de régulation de pression, entraînant un changement dudit effluent du réacteur d'une suspension liquide/solide en un mélange gaz/solide,
d) diriger ledit mélange gaz/solide dans au moins un cyclone épuiseur, où les solides sont séparés et épurés en utilisant seulement du dioxyde de carbone, et les gaz sortent et sont recyclés, et ensuite
e) récupérer lesdits solides comprenant des isomères du sel alcalin de l'acide dicarboxylique, l'alimentation n'ayant pas réagi, le catalyseur et des traces de coke pour une purification supplémentaire.

2. Procédé selon la revendication 1, dans lequel ledit acide dicarboxylique aromatique est l'acide dicarboxylique de 2,6-naphtalène, lesdits solides sortant du réacteur de dismutation comprennent les isomères de sels de dipotassium de l'acide carboxylique du 2,6- et 2,3-naphtalène, le naphtoate de potassium n'ayant pas réagi, le catalyseur et des traces de coke, et le milieu réactionnel est le naphtalène.

3. Procédé selon la revendication 1, dans lequel la suspension liquide/solide sortant du réacteur de dismutation est à une pression supérieure à 4481 kPa (650 psi).

4. Procédé selon la revendication 1, dans lequel dans l'étape (a) la valve fluidique est un té.

5. Procédé selon la revendication 1, dans lequel dans l'étape (a) la deuxième injection du milieu réactionnel est le naphtalène.

6. Procédé selon la revendication 1, dans lequel dans l'étape (a) ledit contrôle de l'écoulement de l'effluent du réacteur est obtenu en l'absence de moyens mécaniques.

7. Procédé selon la revendication 1, dans lequel une chambre de montée séparée n'est pas nécessaire.

8. Procédé selon la revendication 1, dans lequel après la montée de tout le milieu réactionnel liquide, le mélange gaz/solide est reçu directement dans un cyclone de réception.

9. Procédé selon la revendication 1, dans lequel le dioxyde de carbone gazeux peut être éliminé en dissolvant les solides du produit dans de l'eau.

10. Procédé selon la revendication 2, dans lequel tout le naphtalène est monté sans apport de chaleur supplémentaire.

11. Procédé selon la revendication 1, dans lequel il y a deux stades d'épuiseurs.

12. Procédé selon la revendication 11, dans lequel l'épuiseur du premier stade et le cyclone de réception sont combinés en un seul récipient de type cyclone épuiseur.

13. Procédé selon la revendication 12, dans lequel le premier épuiseur se situe au-dessus du second.

14. Procédé selon la revendication 13, dans lequel le CO₂ gazeux est introduit au fond de chaque épuiseur.

15. Procédé selon la revendication 14, dans lequel le CO₂ et certains des solides entraînés sortent des épuiseurs et entrent respectivement dans les cyclones secondaires attachés où les solides supplémentaires sont séparés.

16. Procédé selon la revendication 15, dans lequel les cyclones secondaires sont équipés d'un moyen de retour des solides supplémentaires séparés vers les épuiseurs respectifs.

17. Procédé selon la revendication 16, dans lequel lesdits épuiseurs, l'un au-dessus de l'autre, et les cyclones secondaires attachés, chacun avec un moyen de retour des solides séparés vers les épuiseurs auxquels il est attaché, permettent une récupération maximale des solides, et les solides combinés sortent du second épuiseur.

18. Procédé selon la revendication 3, dans lequel lesdits solides sortant dudit réacteur de dismutation sous la forme d'une suspension liquide/solide sont à une pression comprise entre 5171 à 6550 kPa (750 à 950 psi).

19. Procédé selon la revendication 2, dans lequel ledit produit solide comprenant les sels de dipotassium de l'acide dicarboxylique du naphtalène, les sels de dipotassium de l'acide dicarboxylique du 2,6- et 2,3-naphtalène, le KNA n'ayant pas réagi, le catalyseur, des sous-produits lourds et des traces de coke sortent du dernier épuiseur et sont directement dirigés vers un lavage à l'eau où les sels organiques sont dissous.

20. Procédé selon la revendication 2, dans lequel la vapeur de naphtalène sortant des épuiseurs est recyclée.
